# EUROPEAN PATENT APPLICATION

(11) **EP 4 245 241 A1**
(43) Date of publication of application: **20.09.2023**
(21) Application number: 22161937.2
(22) Date of filing: 14.03.2022
(51) Int. Cl.: A61B 34/10, A61B 18/14

(54) **A SYSTEM FOR THERMAL ABLATION TREATMENT PLANNING**

(71) Applicant: Koninklijke Philips N.V., 5656 AG Eindhoven (NL)
(72) Inventor: ISOLA, Alfonso Agatino, Eindhoven (NL); CIANCARELLA, Martina, Eindhoven (NL); HAUTVAST, Guillaume Leopold Theodorus Frederik, Eindhoven (NL); STROOSMA, Otto, Eindhoven (NL)
(74) Representative: Philips Intellectual Property & Standards

(57) **Abstract**

A system (100) for thermal ablation treatment planning is provided that allows to provide an entry condition (21a, 21b; 22a, 22b) indicative of how a trajectory of a thermal ablation source (50) is to enter a treatment region (60). A configuration space is then provided that comprises, as configurations, a) candidate positions corresponding to candidate trajectories (311a, 311b; 312a, 312b) entering the treatment region in accordance with the provided entry condition, and b) candidate thermal control parameter values (32i, 32ii) for each of the candidate positions. Based on candidate thermal impacts (10a, 10b, 10c, 10d) provided for each of the configurations, a treatment plan is determined that satisfies a thermal impact objective.

## Description

### FIELD OF THE INVENTION

The invention relates to a system, a method and a computer program for thermal ablation treatment planning.

### BACKGROUND OF THE INVENTION

Treating lesions by thermal ablation requires careful planning in order to sufficiently ablate the lesion without unnecessarily damaging healthy tissue. Optimizing thermal ablation treatment plans, however, is a complex problem due to the many variables involved, even assuming perfect navigational capabilities. It is therefore desirable that more efficient means for planning thermal ablation treatments are provided.

### SUMMARY OF THE INVENTION

It is an object of the invention to provide more efficient means for planning thermal ablation treatments.

In a first aspect, the invention relates to a system for thermal ablation treatment planning, wherein the system comprises:
- an entry condition providing unit configured to provide an entry condition indicative of how a trajectory of a thermal ablation source to be planned is to enter a treatment region comprising an ablation target region,
- a configuration space providing unit configured to provide a configuration space comprising, as configurations, a) candidate positions corresponding to a plurality of candidate trajectories of the thermal ablation source through the treatment region and b) candidate thermal control parameter values for each of the candidate positions, wherein the candidate trajectories enter the treatment region in accordance with the provided entry condition,
- a candidate thermal impact providing unit configured to provide candidate thermal impacts generated by the thermal ablation source on its neighborhood in each of the configurations, and
- a treatment plan determining unit configured to determine a thermal ablation treatment plan comprising a) a planned trajectory of the thermal ablation source and b) planned thermal control parameter values along the planned trajectory, wherein the treatment plan is determined based on the candidate thermal impacts provided for each of the configurations such that a predetermined thermal impact objective is satisfied by the treatment plan.

Since the configuration space considered for determining the treatment plan comprises trajectories of the thermal ablation source satisfying the entry condition, the complexity in determining a treatment plan satisfying a predetermined thermal impact objective can be significantly decreased, thereby allowing to significantly increase the efficiency in planning thermal ablation treatments.

A thermal ablation treatment is understood herein to preferably refer to a treatment using heat or cold for ablation, wherein the heat or cold is generated by the thermal ablation source. For instance, heat can be generated in an ablation target, such as a tumor, by radiating microwaves onto and/or into it. For this purpose, an electrode, possibly located at a distal portion of a suitable applicator device, may be brought close to, into contact with or inside the ablation target, wherein the electrode may then be electrically controlled such that it radiates the microwaves.

The entry condition providing unit may be a receiver configured to receive the entry condition and provide the received entry condition for further processing. For instance, the entry condition may be received via a user interface. The entry condition providing unit may also be a memory storing the entry condition for access by other parts of the system, particularly the configuration space providing unit. The entry condition providing unit may also be a processor configured to determine the entry condition based on a data input or an access to stored data.

The configuration space providing unit may particularly be configured to provide a configuration space consisting of, as configurations, a) candidate positions corresponding to a plurality of candidate trajectories of the thermal ablation source through the treatment region and b) candidate thermal control parameter values for each of the candidate positions, wherein the candidate trajectories enter the treatment region in accordance with the provided entry condition. The configuration space providing unit may be a processor coupled to a memory storing a default configuration space comprising all geometrically and/or technically possible configurations, i.e., for instance, candidate positions corresponding to all geometrically and/or technically possible candidate trajectories and all technically possible candidate thermal control parameter values for each of the candidate positions, wherein the processor may be configured to retrieve the default configuration space from the memory, restrict it to those configurations satisfying the entry condition and provide the restricted configuration space for further processing either directly or by storing it in the memory for further access. However, the configuration space providing unit may also be configured to build up the configuration space itself. For instance, the processor may be configured to build up a data structure corresponding to the configuration space, i.e. the configuration space whose configurations already satisfy the entry condition, wherein this data structure may be provided for further processing either directly or by storing it in the memory for further access.

The candidate trajectories of the thermal ablation source will often be linear trajectories that can be followed by an applicator device comprising the thermal ablation source at its distal portion. However, the candidate trajectories could also be curved. The candidate positions corresponding to a given candidate trajectory preferably refer to the points collectively making up the given candidate trajectory. If representing a candidate trajectory in a discretized way, a finite set of points can be identified on it.

The candidate thermal control parameter values, which are candidate values of one or more thermal control parameters, are indicative of a candidate thermal impact, i.e. a thermal impact that would be generated by the thermal ablation source on its neighborhood if it were positioned at a respective candidate position and controlled according to the candidate thermal control parameters. Candidate positions at which the thermal control parameters lead to at least some thermal impact could be considered candidate activation positions, as the thermal ablation source is activated at these positions. A thermal impact generated by the thermal ablation source on a point in its neighborhood is preferably understood herein as referring to a thermal energy transferred to or withdrawn from the point, which may be indicated by an increase or a decrease in temperature.

The candidate thermal impact providing unit can be, for instance, a processor coupled to a memory storing control characteristics of the thermal ablation source and possibly thermal characteristics of the treatment region, wherein the processor may be configured to determine candidate thermal impacts generated by the thermal ablation source on its neighborhood in each of the configurations based on the respective candidate thermal control parameter values and the stored characteristics, and to provide the determined candidate thermal impacts for further processing either directly or by storing it in the memory for further access.

At a given candidate position, and given thermal characteristics in a neighborhood of the candidate position, the candidate thermal control parameters can imply a thermal impact generated by the thermal ablation source in the neighborhood when being at the candidate position. Hence, the configuration space can particularly also be considered as comprising, as configurations, the positions corresponding to the plurality of candidate trajectories and, for each of the positions, the respectively provided candidate thermal impacts for different candidate thermal control parameter values. A thermal impact generated by the thermal ablation source in its neighborhood could also be considered as a local thermal impact. On the other hand, the thermal impact objective satisfied by the determined treatment plan preferably refers to a global thermal impact. A global thermal impact could be indicative of a plurality of local thermal impacts generated during a thermal ablation treatment, and/or a thermal impact evaluated across the whole treatment region.

The treatment plan determining unit can particularly be configured to determine a thermal ablation treatment plan consisting of a) the planned trajectory of the thermal ablation source and b) the planned thermal control parameter values along the planned trajectory. The treatment plan determining unit can be a processor configured to use the candidate thermal impacts provided by the candidate thermal impact providing unit for each of the configurations provided by the configuration space providing unit in order to evaluate each, particularly any subset, of the configurations with respect to the thermal impact objective. In particular, any combination of configurations satisfying the thermal impact objective at least to a predetermined degree can be assumed to form a viable treatment plan.

The planned thermal ablation treatment can, in particular, refer to a percutaneous ablation tumor, particularly cancer, treatment. The thermal ablation source can be part of a thermal ablation modality, particularly of a needle-like applicator, for instance, which may comprise the thermal ablation source at or near its tip. The thermal ablation modality to be used can be chosen, for instance, based on the organ comprising the tumor, the size of the tumor and its location, such as with respect to the organ. Any of a radiofrequency (RF), a focused ultrasound (FU), a microwave (MW), a laser, a cryo-ablation and an irreversible electroporation (IRE) based ablation modality can be chosen, for instance.

The entry condition providing unit can be configured to provide a plurality of entry conditions, wherein the configuration space providing unit can be configured to provide the configuration space such that the candidate trajectories each enter the treatment region in accordance with one of the entry conditions, wherein the treatment plan determining unit can be configured to determine the thermal ablation treatment plan such that it comprises, particularly consists of, a) a plurality of planned trajectories to be followed during treatment and b) planned thermal control parameter values along the planned trajectories. Planning a plurality of trajectories with possibly different entry conditions allows for satisfying more complex thermal impact objects. However, even then it is not necessary to try out all possible entry conditions. Instead, only plausible entry conditions might be tried, wherein plausibility may refer to the judgement of a trained physician relying on his or her experience, for instance. Sequentially following a plurality of planned trajectories can refer to following the trajectories with one or several applicators corresponding to a respective thermal ablation source. Several applicators can be present in the treatment region at the same time. It is understood that the provided entry conditions need not necessarily all be realized in the finally determined treatment plan. However, the finally determined treatment plan preferably comprises no trajectories not satisfying any of the provided entry conditions.

The entry conditions can each comprise a stipulated entry direction, wherein for each of the stipulated entry directions, a plurality of candidate entry points at which the one or more candidate trajectories entering the treatment region in the stipulated entry direction do so can be determined based on the stipulated entry direction and the treatment region. In this way, geometrically complete entry conditions for all candidate trajectories can be obtained. If, as might be preferred, the candidate trajectories correspond to segments of straight lines, these lines are therefore fixed. In particular, the plurality of candidate entry points at which the one or more candidate trajectories entering the treatment region in the stipulated entry direction do so can be determined such that the resulting candidate trajectories intersect the ablation target region. The "stipulated" entry direction need not necessarily all be realized in the finally determined treatment plan. However, the finally determined treatment plan preferably comprises no trajectories not entering the treatment region in any of the stipulated entry directions.

In an example, the stipulated entry directions may be given in terms of unit vectors, the treatment region may be a partial volume of a patient's body and the ablation target region may be a tumor in the partial volume of the patient's body. For instance, the unit vectors may be determined based on a user input successively indicating two points in a 3D-rendered image of the patient. A plane may be defined for each treatment such that it is perpendicular to the respective unit vector and lies outside of the partial volume of the patient's body. On each of these planes, a set of projection points may be defined by virtually projecting the tumor onto the plane against the respective stipulated entry direction, i.e. opposite to the direction of the respective unit vector, wherein the projection points may be defined as all points, possibly grid points of a discrete two-dimensional coordinate system on the respective plane, which lie inside the projection. The projection points can be projected back in the respective stipulated entry direction, i.e. in the direction of the respective unit vector, onto a surface of the partial volume of the patient's body, i.e. preferably the patient's skin, in order to obtain the candidate entry points. Some of the candidate entry points may be subsequently discarded, namely if it is determined that a trajectory entering through the respective entry point in the respective stipulated entry direction would pierce a region in the partial volume of the patient's body that is not supposed to get in contact with the thermal ablation source.

The entry conditions can each comprise a stipulated entry point, wherein for each of the stipulated entry points, a plurality of candidate entry directions in which the one or more candidate trajectories entering the treatment region at the stipulated entry point do so can be determined based on the stipulated entry point and the treatment region. Also in this way, geometrically complete entry conditions for all candidate trajectories can be obtained, wherein if, as might be preferred, the candidate trajectories correspond to segments of straight lines, these lines are therefore fixed. In particular, the plurality of candidate entry directions in which the one or more candidate trajectories entering the treatment region at the stipulated entry point do so can be determined such that the resulting candidate trajectories intersect the ablation target region. The "stipulated" entry points need not necessarily all be realized in the finally determined treatment plan. However, the finally determined treatment plan preferably comprises no trajectories not entering the treatment region in any of the stipulated entry points.

For instance, the stipulated entry points can be provided by detecting radio-opaque marks on the patient's skin, or in response to a user placing a mark virtually on the patient's skin in a 3D-rendered image of the patient. The stipulated entry points can particularly be provided with a predetermined spacing. In this way, the use of a rigid/leading grid template for assisting in the insertion of the thermal ablation probe can be mimicked.

Radio-opaque marks on the patient's skin defining stipulated entry points having a predetermined spacing may also be generated by a user using a flexible radio-opaque grid, wherein the user may generate, particularly "tattoo" with radio-opaque ink, the marks through the holes of the flexible radio-opaque grid.

In case of thermal ablation treatments carried out with a rigid/leading grid template, like e.g. for prostate cancer thermal ablation treatments, the stipulated entry points could be assumed to correspond to where the holes of the rigid/leading grid template reach the surface of the treatment region, such as the patient's skin, for instance. Moreover, a single stipulated entry direction could be assumed that corresponds to the direction orthogonal to the grid template plane, i.e. the direction of its holes.

The candidate thermal control parameter values along the candidate trajectories can be chosen such that the thermal ablation source is only activated inside the ablation target region. In other words, the "active" part of the candidate trajectories can be restricted to the intersection of the candidate trajectories with the ablation target region. In this way, a further decrease in the number of configurations that need to be considered for determining the treatment plan can be achieved.

The candidate thermal control parameter values can indicate activation positions along the respective candidate trajectory at which the thermal ablation source is activated, and a candidate thermal impact of the activated thermal ablation source at the respective activation positions. The indicated thermal impacts can be those provided by the candidate thermal impact providing unit. The thermal impact can be indicated in terms of an ablation zone corresponding to a region receiving thermal energy beyond a predetermined threshold. For instance, the ablation zone can have the shape of an ellipsoid. If the thermal ablation source is elongated, for instance, the ablation zone will typically have the shape of an elongated ellipsoid. The thermal impact can also be defined in terms of an ablation power and an ablation time at a given activation position. For instance, if the thermal ablation source rests at a given activation position, an ablation zone can grow over some time even if the ablation power is left constant.

The candidate thermal control parameter values can indicate a fixed set of activation positions along each of the candidate trajectories and/or a fixed set of candidate thermal impacts. This can further reduce the size of the configuration space and therefore increase the planning efficiency. In particular, the candidate thermal control parameter values can indicate a fixed set of activation positions along each of the candidate trajectories in the ablation target region and/or a fixed set of candidate thermal impacts in the ablation target region. The set of candidate thermal impacts can correspond to a set ablation zone shapes, for instance.

The thermal impact objective to be satisfied by the treatment plan can relate to any of an overall thermal impact on the ablation target region, an overall thermal impact on regions outside the ablation target region, a number of activation positions, and/or a geometric relation of activation positions to the ablation target region. An overall thermal impact on a part, such as a sub-region, of the treatment region can refer to a total amount of thermal energy delivered to or withdrawn from the part of the treatment region, or to a fraction of the part for which an amount of thermal energy delivered or withdrawn is beyond a predetermined threshold. Hence, the overall thermal impact on a part of the treatment region can also refer to a coverage percentage by ablation zones for that part. Regions outside the ablation target region can particularly be critical structures, for which any substantial thermal impact may need to be avoided. A geometric relation of activation positions to the ablation target region can particularly comprise a symmetric arrangement of activation positions in the ablation target region. For instance, a thermal impact objective may be that the overall thermal impact on the ablation target region be maximized, or that the overall thermal impact on regions outside the ablation target region be minimized.

Determining the treatment plan such that the one or more thermal impact objectives are satisfied may correspond to an optimization over all considered configurations. The considered configurations are however, as described above, preferably limited by, for instance, considering only candidate trajectories with certain entry conditions and/or only certain candidate thermal control parameter values.

The thermal impact objective can comprise several sub-objectives, wherein the sub-objectives can be ranked by priority, and wherein, for determining the treatment plan, candidate treatment plans can be compared with respect to the sub-objectives according to their priority. Candidate treatment plans preferably refer to one or more candidate trajectories with associated candidate thermal control parameter values along them, wherein the candidate trajectories are to be sequentially followed during the treatment to be planned while the thermal ablation source is controlled according to the associated candidate thermal control parameter values. Hence, a candidate treatment plan can refer to a set of candidate configurations to be followed during treatment, i.e. a candidate trajectory through configuration space.

Comparing candidate treatment plans with respect to the sub-objectives according to their priority can refer, for instance, to first comparing the candidate treatment plans with respect to the sub-objective with the highest priority, then comparing the candidate treatment plans that satisfy the sub-objective with the highest priority up to a predetermined first tolerance with respect to the sub-objective with the second-highest priority, then comparing the candidate treatment plans that also satisfy the sub-objective with the second-highest priority up to a predetermined second tolerance with respect to the sub-objective with the third-highest priority, etc.

A particular ranking of sub-objectives by priority could be as follows: 1) Maximum target coverage, 2) minimum coverage of critical structures, 3) minimum healthy tissue coverage percentage, 4) minimum number of ablation zones, 5) ablation symmetry, i.e. providing ablation positions that are symmetric with respect to the ablation target region. A coverage may refer to a geometric coverage by ablation zones.

The determined treatment plan satisfying the thermal impact objective can be modified by removing ablation events that can be removed without generating any part in the ablation target region that receives a thermal impact below a predetermined threshold. An ablation event is understood as an ablation carried out by the thermal ablation source along a planned trajectory. In other words, therefore, the determined treatment plan satisfying the thermal impact objective can be modified by removing activation positions along the planned trajectories that can be removed without generating any part in the ablation target region that receives a thermal impact below a predetermined threshold. For instance, an activation position can be removed when the associated planned ablation zone is fully included in, i.e. covered by, one or more other planned ablation zones.

The entry condition providing unit can be configured to provide the entry conditions based on past treatment plans and/or other clinical data. This allows for a fully automatic determination of treatment plans.

The system can comprise an artificial intelligence providing unit for providing an artificial intelligence that has been trained to provide output entry conditions of thermal ablation treatment plans satisfying a thermal impact objective if it is provided with a three-dimensional input image of an input region comprising an input ablation target region, wherein the entry condition providing unit can be configured to provide the artificial intelligence with a three-dimensional image of the treatment region comprising the thermal ablation target region to be treated, and to provide, as the entry conditions to be used for the treatment to be planned, the output entry conditions provided by the artificial intelligence. The artificial intelligence can comprise a machine learning architecture, such as an artificial neural network, particularly a convolutional neural network, for instance.

The entry condition providing unit can be configured to a) determine a length of intersection of at least a subset of candidate trajectories entering the treatment region according to at least a subset of all possible entry conditions with the ablation target region, wherein the entry conditions can be provided based on the determined lengths of intersection. For instance, those entry conditions may be preferred that lead to higher lengths of intersection. The entry condition providing unit may also be configured to b) determine an ablation target distance for at least a subset of candidate trajectories entering the treatment region according to at least a subset of all possible entry conditions, the ablation target distance being a length of the respective candidate trajectory from entering the treatment region to reaching the ablation target region, wherein the entry conditions can be provided based on the ablation target distances. For instance, those entry conditions may be preferred that lead to lower ablation target distances. Providing the entry conditions based on lengths of intersection with the ablation target region and/or based on ablation target distances can lead to a configuration space comprising candidate trajectories with a higher chance of leading to a satisfactory treatment plan, thereby further increasing the efficiency of the planning process.

A further aspect relates to an apparatus for thermal ablation treatment planning, wherein the apparatus comprises:
- a display device for displaying an image of a treatment region,
- an input receiving unit for receiving user input with respect to the image, and
- the system as defined in any of claims 1 to 12,
wherein the entry condition providing unit is configured to provide the entry conditions based on a user input with respect to the image and/or wherein the display device is configured to visualize the treatment plan in the image.

A further aspect relates to a method for thermal ablation treatment planning, wherein the method comprises:
- providing an entry condition indicative of how a trajectory of a thermal ablation source to be planned is to enter a treatment region comprising an ablation target region,
- providing a configuration space comprising, as configurations, a) candidate positions corresponding to a plurality of candidate trajectories of the thermal ablation source through the treatment region and b) candidate thermal control parameter values for each of the candidate positions, wherein the candidate trajectories enter the treatment region in accordance with the provided entry condition,
- providing candidate thermal impacts generated by the thermal ablation source on its neighborhood in each of the configurations, and
- determining a thermal ablation treatment plan comprising a) a planned trajectory of the thermal ablation source and b) planned thermal control parameter values along the planned trajectory, wherein the treatment plan is determined based on the candidate thermal impacts provided for each of the configurations such that a predetermined thermal impact objective is satisfied by the treatment plan.

A further aspect relates to a method for user-oriented thermal ablation treatment planning, wherein the method comprises:
- displaying an image of a treatment region,
- receiving user input with respect to the image, and
- the steps of the method described so far,
wherein the entry conditions are provided based on a user input with respect to the image and/or wherein the treatment plan is visualized in the image.

A further aspect relates to a computer program for thermal ablation treatment planning, wherein the program comprises program code means for causing the system as defined in any of claims 1 to 12 to execute the method as defined in claim 14.

A further aspect relates to a computer program for thermal ablation treatment planning, wherein the program comprises program code means for causing the apparatus as defined in claim 13 to execute the user-oriented method as defined above if the program is executed on a computer controlling the apparatus.

It shall be understood that the system of claim 1, the apparatus of claim 13, the method of claim 14 and the computer program of claim 15 have similar and/or identical preferred embodiments, in particular as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily a system for thermal ablation treatment planning,
Figs. 2A and 2B show schematically and exemplarily candidate trajectories of two different configuration spaces,
Fig. 3 shows schematically and exemplarily candidate thermal control parameter values leading to different candidate thermal impacts of a thermal ablation source on its neighborhood,
Figs. 4A to 4F illustrate schematically and exemplarily different configurations of activation positions and ablation zones along a candidate trajectory through an ablation target region,
Fig. 5 shows schematically and exemplarily intersections of candidate trajectories with the ablation target region,
Figs. 6A to 6G schematically and exemplarily illustrate particular steps that can be taken to arrive at a treatment plan,
Figs. 7A and 7B show schematically and exemplarily a possible user interface providable on a display of an apparatus comprising a system as shown in Fig. 1,
Fig. 8 shows schematically and exemplarily a method for thermal ablation treatment planning, and
Fig. 9 shows schematically and exemplarily a flowchart summarizing particular elements.

### DETAILED DESCRIPTION OF EMBODIMENTS

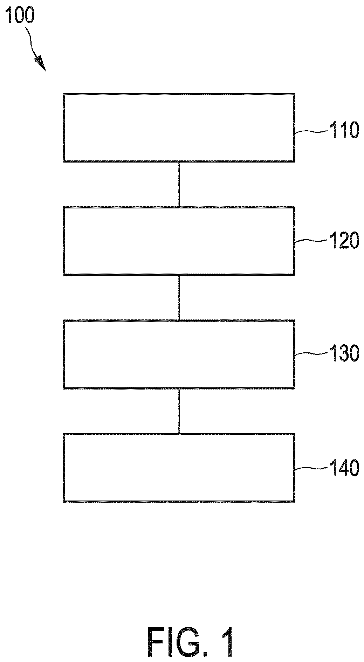

Fig. 1 shows schematically and exemplarily a system 100 for thermal ablation treatment planning that comprises an entry condition providing unit 110, a configuration space providing unit 120, a candidate thermal impact providing unit 130, and a treatment plan determining unit 140.

The entry condition providing unit 110 is configured to provide an entry condition 21a, 21b; 22a, 22b that is indicative of how a trajectory of a thermal ablation source 50 to be planned is to enter a treatment region 60 comprising an ablation target region 70.

The configuration space providing unit 120 is configured to provide a configuration space, wherein the configuration space comprises, as configurations, a) candidate positions corresponding to a plurality of candidate trajectories 311a, 311b; 312a, 312b of the thermal ablation source 50 through the treatment region 60, and b) candidate thermal control parameter values 32i, 32ii for each of the candidate positions, wherein the candidate trajectories 311a, 311b; 312a, 312b enter the treatment region 60 in accordance with the provided entry condition 21a, 21b; 22a, 22b, i.e. the entry condition that has been provided by the entry condition providing unit 110.

The configuration space could be thought of as a set of pairs of a) coordinates of the respective candidate position and b) corresponding candidate thermal control parameter values 32i, 32ii. Geometrically, the configuration space could be thought of as comprising the candidate positions corresponding to the plurality of candidate trajectories 311a, 311b; 312a, 312b of the thermal ablation source 50 through the treatment region 60, i.e. points along the trajectories, and labels comprising corresponding candidate thermal control parameter values 32i, 32ii by which each of the points is labeled.

The candidate thermal impact providing unit 130 is configured for providing candidate thermal impacts 10a, 10b generated by the thermal ablation source 50 on its neighborhood in each of the configurations, i.e. in each of the configurations in the configuration space provided by the configuration space providing unit 120. The candidate positions, the candidate thermal control parameter values 32i, 32ii and the candidate thermal impacts are understood as possible, or virtual, positions, thermal control parameter values and thermal impacts, respectively. They are not yet, at least not all, the actual positions, actual thermal control parameter values and actual thermal impacts to be delivered in the planned treatment, but provide a basis for determining the treatment plan.

The treatment plan determining unit 140 is configured to determine a thermal ablation treatment plan, wherein the thermal ablation treatment plan comprises a) a planned trajectory 41a, 41b of the thermal ablation source 50 and b) planned thermal control parameter values along the planned trajectory 41a, 41b. The treatment plan determining unit 140 is configured to determine the treatment plan based on the candidate thermal impacts 10a, 10b provided for each of the configurations, i.e. the configurations in the configuration space provided by the configuration space providing unit 120 such that a predetermined thermal impact objective is satisfied by the treatment plan.

The treatment plan determining unit 140 can assume the form of a mathematical solver that searches for the best treatment plan comprising the best set of trajectories entering the treatment region 60, like the skin of a patient, for instance, and ablation settings corresponding to particular thermal control parameter values along the trajectories. The treatment plan determining unit 140 can be beneficial in simplifying a treatment planning process, in increasing the ablation target region coverage quality according to the treatment plan, in standardizing the whole planning process further, and in making the whole planning process more efficient.

It is clear that the entry condition providing unit 110, the configuration space providing unit 120, the candidate thermal impact providing unit 130 and the treatment plan determining unit 140 can carry out their respective operations by reference to a representation of the treatment region 60 and the ablation target region 70, which can be, for instance, provided in terms of a three-dimensional image, particularly a segmented image. Specifically, if the treatment region 60 corresponds to a partial volume of a patient's body and the ablation target region 70 corresponds to a tumor in the partial volume, the representation can be given in terms of a three-dimensional medical image of the partial volume of the patient's body and the tumor therein, wherein the tumor and possibly also other anatomical structures may be segmented in the image. The tumor can particularly be a cancer lesion that is to be destroyed by the thermal ablation treatment. If, for instance, the cancer is a renal cancer, the imaged partial volume of the patient may correspond to the abdomen of the patient. Optionally, the system 100 further comprises a treatment region representation providing unit for providing, i.e. which is configured to provide, a representation of the treatment region 60 comprising the ablation target region 70, wherein the representation may be a three-dimensional image, for instance.

Since the treatment plan is determined such that a predetermined thermal impact objective is satisfied by the treatment plan, it is also clear that the determined treatment plan preferably depends on the predetermined thermal impact objective. Optionally, the system 100 further comprises a thermal impact objective providing unit for providing, i.e. which is configured to provide, the predetermined, i.e. a predeterminable, thermal impact objective. The thermal impact objective can be predetermined and/or provided based on clinical prescriptions. It may also be considered as a treatment protocol objective.

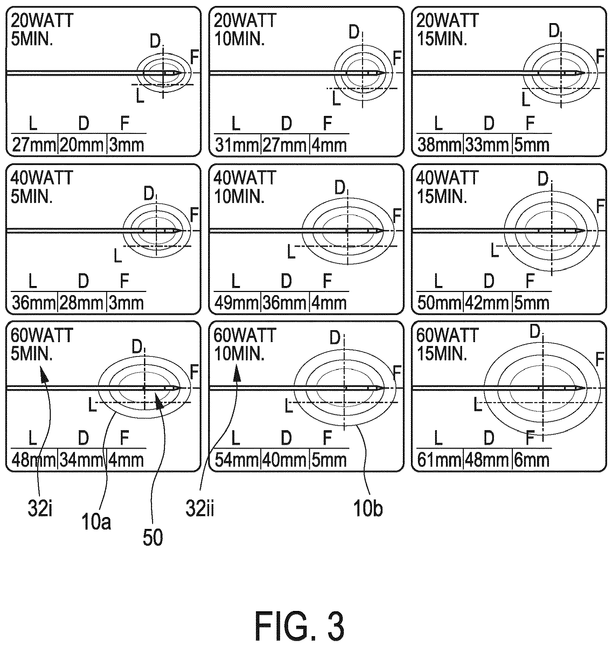

The candidate thermal impact providing unit 130 may provide the candidate thermal impacts 10a, 10b generated by the thermal ablation source 50 on its neighborhood in any of the configurations based on a result of a calibration, wherein the calibration may correspond to a measurement or simulation of thermal impacts in neighborhoods of the actual or simulated thermal ablation source 50 for a plurality of thermal control parameter values 32i, 32ii. Hence, the thermal ablation source 50 is preferably a commissioned device for which it is known, at least roughly or in an idealized situation, which ablation characteristics follow from given ablation settings of the device. This will be further explained below with respect to Fig. 3.

The treatment plan determining unit 140 may be configured to determine the treatment plan based on a plurality of further kinds of data, which may relate, for instance, to treatment workflow preferences, such as whether pullbacks of the thermal ablation source 50 should be considered/allowed. The system 100 may then comprise further providing units for providing such further kinds of data.

The entry condition providing unit 110 may be configured to provide the entry condition 21a, 21b; 22a, 22b based on an input provided by a user of the system 100, or an apparatus comprising the system 100, via a suitable user interface. The user may be a physician that seeks assistance in planning a thermal ablation treatment.

The system 100 preferably provides, as an output, an optimal treatment plan corresponding to the thermal ablation source 50 being inserted into the treatment region 60, such as via the skin of a patient, according to the provided entry condition 21a, 21b; 22a, 22b and optimally delivering thermal impacts along its way through the treatment region 60. The treatment plan can be determined for a plurality of thermal ablation sources, in which case the entry condition providing unit 110 can be configured to provide a plurality of entry conditions 21a, 21b; 22a, 22b, wherein the configuration space providing unit 120 can be configured to provide the configuration space such that the candidate trajectories 311a, 311b; 312a, 312b each enter the treatment region 60 in accordance with one of the entry conditions 21a, 21b; 22a, 22b, wherein the treatment plan determining unit 140 can be configured to determine the thermal ablation treatment plan such that it comprises a) a plurality of planned trajectories to be followed during treatment and b) planned thermal control parameter values along the planned trajectories. The plurality of planned trajectories can be followed sequentially or at least partially simultaneously, particularly by different ones of the plurality of thermal ablation sources. For instance, two planned trajectories can be followed sequentially by a single ablation source or at least partially simultaneously by two ablation sources.

Generally, the task carried out by the treatment plan determining unit 140 would be relatively complex if an arbitrary configuration space were considered, particularly a configuration space comprising a relatively high density of candidate positions, as may particularly be relevant when representing the treatment region 60 with a relatively high degree of detail, such as by an image with a relatively high resolution. This is because the number of candidate treatment plans to be evaluated by the treatment plan determining unit 140 with respect to the predetermined thermal impact objective would then be relatively high. However, since a configuration space is considered that is restricted to trajectories satisfying the provided one or more entry conditions, the set of configurations that need to be considered by the treatment plan determining unit 140 for evaluation with respect to the thermal impact objective can be decreased, such that the treatment plan can be determined in acceptable time frames.

The complexity of the task to be carried out by the treatment plan determining unit 140 caused by the cardinality of a generic configuration space becomes even more relevant if the treatment to be planned comprises a plurality of planned trajectories 41a, 41b to be sequentially followed during the treatment, and planned thermal control parameter values along the planned trajectories 41a, 41b, in which case usually a plurality of entry conditions 21a, 21b; 22a, 22b will be provided, wherein the candidate trajectories 311a, 311b; 312a, 312b each enter the treatment region 60 in accordance with one of the entry conditions 21a, 21b; 22a, 22b.

The system 100 can be particularly useful in improving the workflow of thermal ablation treatment planning if the treatment to be planned is to be carried out in a freehand manner. When carrying out a thermal ablation treatment in a freehand manner, i.e. without systems or devices like leading grid templates, for instance, the planning task is usually considered a relatively difficult problem due to the relatively high number of degrees of freedom involved.

The number of degrees of freedom is reflected in the cardinality of the configuration space considered, wherein the cardinality is understood as referring to the number of elements in the configuration space, the elements corresponding to the pairs of a) candidate positions corresponding to the plurality of candidate trajectories 311a, 311b; 312a, 312b of the thermal ablation source 50 through the treatment region 60 and b) candidate thermal control parameter values 32i, 32ii for each of the candidate positions. Under some circumstances, namely when not a single optimal treatment plan exists but a whole solution space of acceptable treatment plans, even the cardinality of the solution space can become quite large, such that it may become difficult to pick a treatment plan to actually follow from the solution space.

As will also become clear in the following, the system 100 may be realized to function semi-automatically or fully automatically, particularly depending on how the one or more entry conditions 21a, 21b; 22a, 22b are provided.

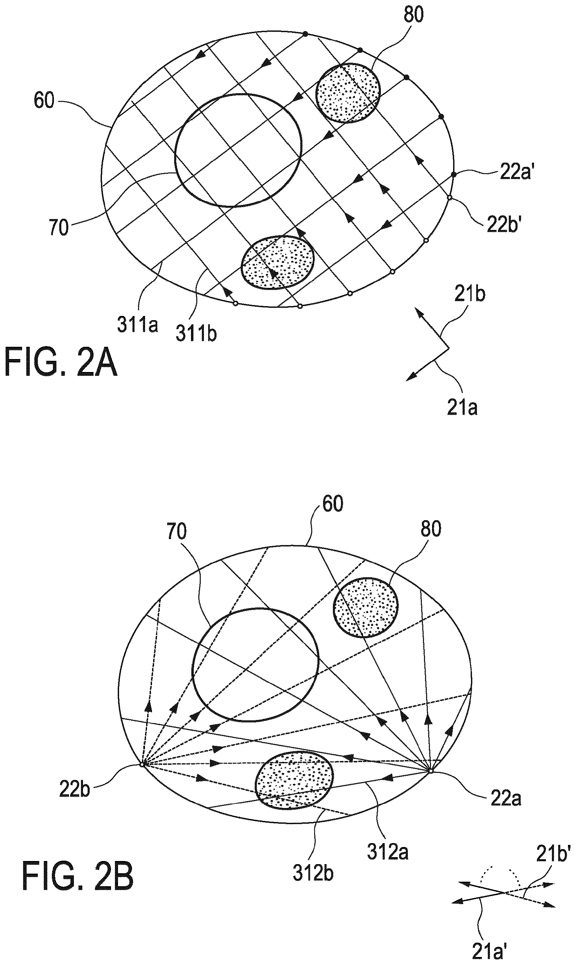

Figs. 2A and 2B schematically and exemplarily show a respective configuration space provided by the configuration space providing unit 120 for the same treatment region 60 comprising the same ablation target region 70. The treatment region 60 further comprises two risk regions 80 corresponding to an anatomical structure whose ablation would constitute a high medical risk. The risk regions 80 could therefore also be thought of as no-go zones for the thermal ablation source 50 and/or any insertion device of which it is a part. Any planned trajectory should avoid the risk regions 80. For instance, the risk regions 80 could correspond to particular organs, a ureter, the colon, vessels, nerves, muscles etc., while the ablation target region 70 could correspond to a renal cancer lesion. The treatment region 60, which could correspond to an abdomen of a patient, is represented in Figs. 2A and 2B as it would also appear in an axial slice of a three-dimensional medical image.

As illustrated schematically and exemplarily by Fig. 2A, the entry conditions 21a, 21b; 22a, 22b can each comprise a stipulated entry direction 21a, 21b, wherein for each of the stipulated entry directions 21a, 21b, a plurality of candidate entry points 22a', 22b' at which the one or more candidate trajectories 311a, 311b that enter the treatment region 60 in the stipulated entry direction 21a, 21b do so can be determined based on the stipulated entry direction 21a, 21b and the treatment region 60. For instance, all directed lines in the provided stipulated entry direction 21a, which are necessarily parallel to each other, that have a predetermined distance to each other are first considered, wherein then, for all of these directed lines, intersections with the treatment region 60 are determined. These intersections correspond to the candidate trajectories 311a through the treatment region 60. Similarly, candidate trajectories 311b can be determined based on the provided stipulated entry direction 21b. Based on the candidate trajectories 311a, 311b, the candidate entry points 22a', 22b' can be determined as those points where the respective candidate trajectories 311a, 311b enter the treatment region 60. Hence, the candidate entry points 22a', 22b' correspond to intersections of the directed lines in the respective candidate direction 21a, 21b with a surface or boundary of the treatment region 60, which may correspond, for instance, to a patient's skin.

Fig. 2B illustrates schematically and exemplarily that the entry conditions 21a, 21b; 22a, 22b can also each comprise a stipulated entry point 22a, 22b, wherein for each of the stipulated entry points 22a, 22b, a plurality of candidate entry directions 21a', 21b' in which the one or more candidate trajectories 312a, 312b entering the treatment region 60 at the stipulated entry point 22a, 22b do so can be determined based on the stipulated entry point 22a, 22b and the treatment region 60. For instance, all directed lines entering the treatment region 60 through the stipulated entry point 22a may first be considered that have a predetermined angular distance to each other, wherein then an intersection of each of these directed lines with the treatment region 60 may be determined. The candidate trajectories 312a, 312b could then correspond to these intersections. Similarly, candidate trajectories 312b can be determined based on the provided stipulated entry point 22b. The directions of the thus generated candidate trajectories 312a, 312b, which correspond to the respective directions of the initially considered directed lines that cross the respective stipulated entry points 22a, 22b in the direction of the interior of the treatment region 60, are assumed as the candidate directions 21a', 21b'.

Fig. 3 shows schematically and exemplarily how the thermal ablation source 50 can be calibrated, and thus which kind of data can be used by the candidate thermal impact providing unit 130 for providing the candidate thermal impacts 10a, 10b generated by the thermal ablation source 50 on its neighborhood in each of the configurations in configuration space. This kind of data can, for instance, be provided by a supplier of the thermal ablation source. Fig. 3 shows a plurality of simulation results carried out for the thermal ablation source 50 with different thermal control parameter values 32i, 32ii. The thermal control parameters considered in this example correspond to an ablation power and an ablation time, wherein, as seen in Fig. 3, the ablation power values are given in watts and the ablation time values are given in minutes. In the example shown, the thermal ablation source 50 corresponds to a distal portion of a needle-shaped thermal applicator, which may also be referred to as an ablation probe. Depending on the values 32i, 32ii of the thermal control parameters according to which the thermal applicator, particularly the thermal ablation source 50, is controlled, different thermal impacts generated in a neighborhood of the thermal ablation source 50 are observed according to the shown simulation results. The generated thermal impact decreases with increasing distance from the thermal ablation source 50. Since, in this case, the thermal ablation source 50 at the distal portion of the thermal applicator is elongated, regions receiving equal or similar thermal impact also have an elongated, particularly ellipsoidal shape.

For each combination of thermal control parameter values 32i, 32ii, an ablation zone may be defined as a geometric characteristic for the corresponding thermal impact generated. The corresponding generated thermal impact may therefore also be identified with the ablation zone. The ablation zone may be defined as the respective region in the neighborhood of the thermal ablation source 50 which receives a thermal energy beyond a predefined threshold. Due to the elongated thermal ablation source 50, also the ablation zones will generally be elongated, particularly ellipsoidal in shape. In Fig. 3, for each of the simulation results, a length (L) and a diameter (D) of the respective ablation zones generated is given, as well as a forward distance (F) indicating how far the ablation zone extends beyond the thermal ablation source 50, or a tip of the thermal applicator, in an axial direction of the applicator. The forward distance therefore indicates a position of the ablation zone with respect to the thermal ablation source 50 and/or a tip of the applicator, while the length and the diameter of the ablation zone indicate its intrinsic geometry.

Simulation results as those indicated in Fig. 3 assume models of heating and/or cooling in the neighborhood of the thermal ablation source. It is understood that these models may not accurately apply to the tissue in the actual treatment region 60 for which the thermal ablation treatment is to be planned. Hence, it is to be expected that the thermal impacts generated by the thermal ablation source 50, and therefore also the shape of the ablation zones, will differ from the simulation results in real applications. The thermal impacts, particularly the ablation zone shapes, shown in Fig. 3 may therefore also be understood as "expected" thermal impacts or ablation zones, respectively. Factors that might cause deviations in the results of realistic ablation events as compared to simulations include the environment of the ablation target region 70, such as a tumor's environment, microperfusion within the tissue, a proximity of the position of the ablation event to large blood vessels, etc. Generally, the thermal impact generated by the thermal ablation source 50 also depends on the type of thermal ablation source 50. Fig. 3, for instance, shows simulation results for a microwave ablation probe comprising an electrode at its distal portion as a thermal ablation source 50. The simulations have been carried out using a finite element analysis.

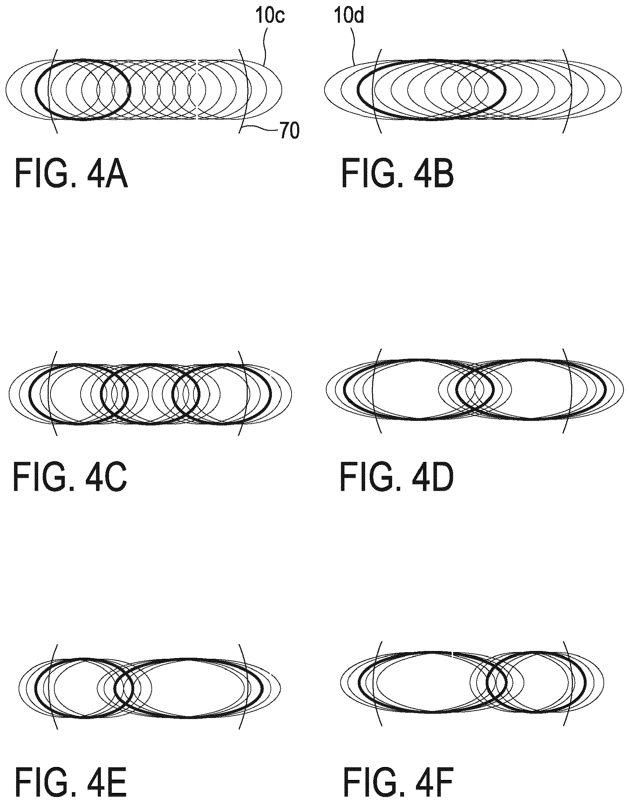

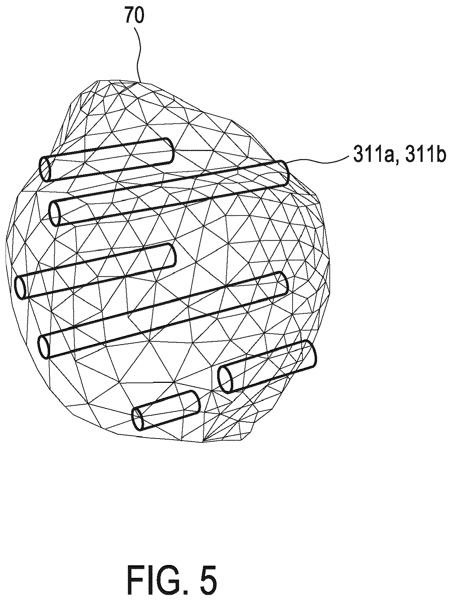

Figs. 4A to 4F illustrate schematically and exemplarily that the candidate thermal control parameter values 32i, 32ii along the candidate trajectories 311a, 311b; 312a, 312b can be chosen by the configuration space providing unit 120 such that the thermal ablation source 50 is only activated inside the ablation target region 70. This may effectively be understood as cropping or restricting the candidate trajectories 311a, 311b; 312a, 312b to their intersections with the ablation target region 70, as will also be described with reference to Fig. 5. In this way, the size of the configuration space considered for determining the treatment plan can be further decreased, such that even less configurations need to be considered for determining the treatment plan. Figs. 4A to 4F focus on the ablation target region 70, wherein its boundary towards the surrounding part of the treatment region 60 is highlighted.

Figs. 4A to 4F also illustrate schematically and exemplarily that the candidate thermal control parameter values 32i, 32ii can indicate activation positions along the respective candidate trajectory 311a, 311b; 312a, 312b at which the thermal ablation source 50 is activated, and a thermal impact 10a, 10b of the activated thermal ablation source at the respective activation positions. In particular, the activation positions can all lie within the target ablation region 70, and along a single candidate trajectory 311a, 311b; 312a, 312b. The trajectory 311a, 311b; 312a, 312b itself is not indicated in Figs. 4A to 4F. It is immediately appreciated from Figs. 4A to 4F that, even for a single candidate trajectory that is, in addition, intersecting the ablation target region 70, the number of configurations to be considered can become quite large due to range of possible thermal control parameter values 32i, 32ii along the trajectory, including the choice of activation positions.

A first class of candidate trajectories 311a, 311b; 312a, 312b with associated thermal ablation control parameter values along them may be determined by the configuration space providing unit 120 such that they comprise only a single activation position with fixed thermal control parameter values 32i, 32ii. Such trajectories are shown in Figs. 4A and 4B, which only differ from each other in the magnitude of the thermal control parameter values 32i, 32ii at the respective single activation position and hence the corresponding thermal impact 10c, 10d indicated by the shown ablation zone shapes. As indicated in Figs. 4A and 4B, the single activation position will have to be selected out of a plurality of candidate activation positions along the respective candidate trajectory 311a, 311b; 312a, 312b.

Figs. 4C and 4D illustrate candidate trajectories 311a, 311b; 312a, 312b with associated candidate thermal control parameter values 32i, 32ii along them for the case of a plurality of activation positions along a single candidate trajectory 311a, 311b; 312a, 312b, particularly three and two activation positions, respectively. The candidate thermal control parameter values 32i, 32ii at the different activation positions shown in Figs. 4C and 4D are the same, and correspond to the ones from Figs. 4A and 4B, respectively, which can be appreciated from the indicated ablation zone shapes.

The configuration space providing unit 120 can also consider different thermal control parameter values 32i, 32ii at activation positions along a single candidate trajectory 311a, 311b; 312a, 312b. This is the case illustrated in Figs. 4E and 4F, in which the different ablation zone shapes already shown in Figs. 4A to 4D are mixed along a single candidate trajectory 311a, 311b; 312a, 312b.

As in Figs. 4A and 4B, also Figs. 4C to 4F show that the plurality of activation positions, in this case two or three, can be located at different positions, i.e. candidate activation positions, along the respective candidate trajectory 311a, 311b; 312a, 312b. It can also be understood from Figs. 4C to 4F that reference activation positions may be defined in predetermined intervals along the intersection of a respective candidate trajectory 311a, 311b; 312a, 312b with the ablation target region 70, wherein the reference activation positions may be considered in terms of an offset with respect to a respective reference activation position. If the considered offsets are restricted to lie, for instance, within half of the respective predetermined intervals in forward and backward direction, considering equivalent configurations multiple times, and therefore redundancy, can be avoided. For instance, in Fig. 4C, three reference activation positions are defined, wherein for each reference activation position, two activation positions are considered in front of the respective reference activation position and two activation positions are considered behind the respective reference activation position. The considered offsets can be integer multiples of a unit offset, or can be chosen to be distributed in a non-uniform manner.

The candidate thermal control parameter values 32i, 32ii can indicate a fixed set of activation positions along each of the candidate trajectories 311a, 311b; 312a, 312b and/or a fixed set of candidate thermal impacts 10a, 10b, 10c, 10d. This can further reduce the number of configurations to be considered in determining the treatment plan and can therefore further increase planning efficiency. For instance, the activation positions can be stipulated to lie at equidistant positions along the candidate trajectories 311a, 311b; 312a, 312b, wherein the number of activation positions along the candidate trajectories 311a, 311b; 312a, 312b may additionally be limited. More particularly, it can be stipulated that no more than one, two or three activation positions should exist along each candidate trajectory 311a, 311b; 312a, 312b within the ablation target region 70, as illustrated in Figs. 4A to 4F. Fixing a set of candidate thermal impacts 10a, 10b, 10c, 10d can particularly correspond to stipulating a limited number of possible ablation zones, such as ablation zone sizes and/or shapes, for instance. More particularly, the set of considered thermal impacts, particularly ablation zones, along each candidate trajectory within the ablation target region 70 can be limited, such as to one or two types 10c, 10d, as illustrated in Figs. 4A to 4F.

It is understood that the configurations shown in Figs. 4A to 4F would need to be considered for each candidate trajectory 311a, 311b; 312a, 312b, i.e. also for candidate trajectories 311a, 311b; 312a, 312b intersecting the ablation target region 70 at different heights and/or at different angles. Hence, the total number of configurations to be considered for determining the treatment plan can become huge, for example exceed 10,000, especially when dealing with a relatively large ablation target region 70, several types of relatively small ablation target regions 70, multiple stipulated entry directions 21a, 21b and a relatively high entry point sampling density. Due to the several factors involved, and their possible variations, the time needed for determining the treatment plan can vary greatly.

Fig. 5 shows schematically and exemplarily the intersections of different candidate trajectories 311a, 311b having the same stipulated entry direction 21a, 21b with the ablation target region 70. The candidate trajectories 311a, 311b are illustrated in Fig. 5 as tubular path segments in order to indicate an actual, i.e. realistic, extension of a thermal applicator comprising the thermal ablation source 50, such as a needle, when following the respective candidate trajectories 311a, 311b. The path segments shown in Fig. 5 are generated by tracking candidate trajectories 311a, 311b entering the surface or boundary of the treatment region 60 at respective entry points 22a', 22b' determined for a stipulated entry direction 21a, 21b, which could also be regarded as a projection of the stipulated direction 21a, 21b onto the segmented treatment region 60, and, in this case, by pruning the tracked trajectories, or projected directions, to the segments intersecting the ablation target region 70 in order to prevent an intersection with no-go regions. In this pruning process, also any offset of ablation zones with respect to the tip of the thermal applicator comprising the thermal ablation source 50 may be considered. In order to obtain the final set of candidate path or trajectory segments, i.e. the one on which the determination of the treatment plan can be carried out, the segments intersecting the ablation target region 70 may further be shortened in order to take into account a length of the thermal applicator and thereby prevent planning ablation events at positions that cannot actually be reached.

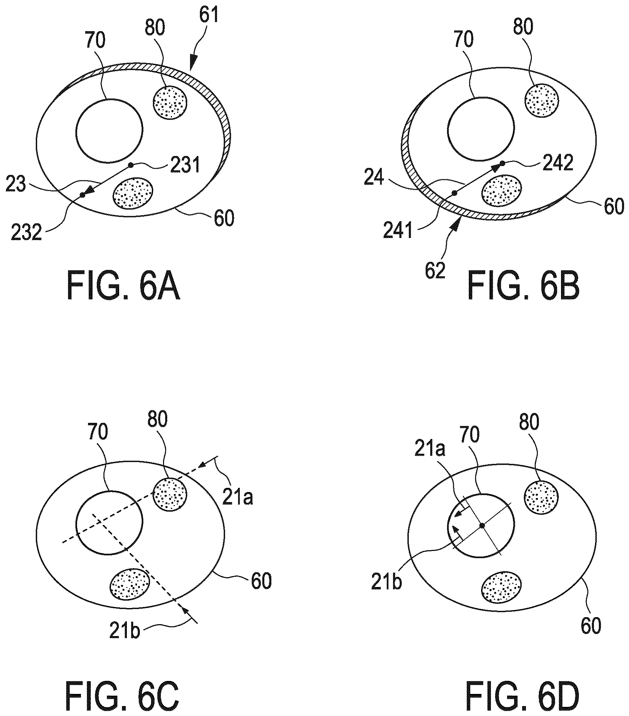

Figs. 6A to 6G schematically and exemplarily illustrate steps taken for thermal ablation treatment planning. According to Figs. 6A to 6G, one or more stipulated entry directions 21a, 21b are provided as entry conditions. Moreover, Figs. 6A to 6G illustrate that the stipulated entry directions 21a, 21b can be provided based on a user input, such as manually providable in a semi-automatic treatment planning process by a clinical expert via an indication tool comprising a user interface.

Figs. 6A and 6B show how the planning process can be initialized through the entry condition providing unit 110. For initialization, a user can, for instance, indicate a desired entry direction 21a, 21b by freely indicating two points 231, 232; 241, 242 in a representation of the treatment region 60, and possibly a region surrounding the treatment region 60, wherein the representation may particularly correspond to a medical image. The indicated two points 231, 232; 241, 242, and the order in which they have been indicated, already imply a user-given stipulated entry direction 21a, 21b, and thus also a side 61, 62 of the treatment region 60, such as a side of the body of a patient to be treated, from which the thermal ablation applicator should be inserted into the treatment region 60 during ablation treatment. The two points 231, 232; 241, 242 can be indicated, for instance, by clicking on a screen showing the medical image.

When representing the two points 231, 232; 241, 242 by three-dimensional vectors *̅P̅*̅₁ and *̅P̅*̅₂, wherein the point corresponding to *̅P̅*̅₁ is indicated first and the point corresponding to *̅P̅*̅₂ is indicated afterwards, then the corresponding unit direction vector can be automatically computed as *P̂*₂₁ = (*̅P̅*̅₂ - *̅P̅*̅₁)/|*̅P̅*̅₂ -*̅P̅*̅₁|.

It does not matter where the user indicates the two points 231, 232; 241, 242 as long as their spatial relationship remains the same, since then the indicated direction 21a, 21b, and thus the computed corresponding unit direction vector, will not change. Accordingly, the user can freely shift the indicated unit direction vectors, or in fact possibly displayed arrows 23, 24 connecting the indicated point pair 231, 232; 241, 242 on the representation of the treatment region 60, without thereby changing the indicated desired entry direction 21a, 21b in which the ablation applicator is to be inserted. This can help a clinician if, for instance, he/she would like to infer the desired entry direction 21a, 21b from a first part in a medical image, but would then like to conclude what this means for a second part of the medical image, or if his/her indication in the first part of the medical image is hardly visible due to particular anatomical structures visualized in the first part, while the indication of the desired entry direction 21a, 21b is more easily visible in the second part of the medical image.

Based on a unit direction vector determined based on the user input, thereby determining a stipulated entry direction 21a, 21b to be provided by the entry condition providing unit 110, candidate trajectories 311a, 311b to be considered for treatment plan optimization by the treatment plan determining unit 140 are determined by the configuration space providing unit 120, as will be subsequently described with reference to Figs. 6C to 6G.

Assuming that, as indicated in Fig. 6C, the user has indicated two desired, i.e. stipulated, entry directions 21a, 21b, such as by sequentially clicking on four points in an image of the treatment region 60, for instance, thereby determining two corresponding unit vectors *̅v̅₁̅*̅, *̅v̅₂̅*̅, these two unit vectors can be shifted to an origin *̅r̅₀̅*̅ serving as a reference point for the further procedure. The reference point *̅r̅₀̅*̅ corresponds to a point in three-dimensional space. For instance, the reference point *̅r̅₀̅*̅ could be chosen to be positioned within the ablation target region 70, particularly at a mesh centroid of a mesh representing, in a discretized manner, the ablation target region 70. Alternatively, for instance, the reference point *̅r̅₀̅*̅ could be chosen to lie at a geometrical barycenter of one or more ablation target regions 70. If there is more than one ablation target region 70, hence, the reference point *̅r̅₀̅*̅ can be positioned at a corresponding common geometrical barycenter of the more than one ablation target regions. It is understood that the procedure generalizes to any number of user-defined entry directions 21a, 21b, and hence corresponding unit vectors.

Fig. 6D shows the reference point *̅r̅₀̅*̅ as a dot within the ablation target region 70. In three-dimensional space, each of the unit vectors *̅v̅₁̅*̅, *̅v̅₂̅*̅; uniquely defines a respective plane, namely the plane to which it is normal. A vector being normal to a plane in a three-dimensional space means that the vector is orthogonal to all vectors lying in the plane. Since, moreover, the unit vectors *̅v̅₁̅*̅, *̅v̅₂̅*̅ have been shifted to the reference point *̅r̅₀̅*̅, two planes intersecting in a line through the reference point *̅r̅₀̅*̅ can be uniquely determined, wherein each of these planes is normal to a respective one of the previously determined unit vectors *̅v̅₁̅*̅, *̅v̅₂̅*̅. This is illustrated in Fig. 6D, in which the planes appear as a cross through the origin defined by the reference point *̅r̅₀̅*̅ due to the representation of the treatment region 60 corresponding to an axial slice through the treatment region 60. In Fig. 6D, the unit vectors *̅v̅₁̅*̅. *̅v̅₂̅*̅ have been shifted again away from the origin for illustrative purposes. Again, it is understood that any number of user-defined directions 21a, 21b and corresponding unit vectors could be determined, in which case a corresponding number of planes could be defined. It is also understood that the planes, as well as the unit vectors, are virtual entities, i.e. not actually present in the real treatment region 60, but only in its representation thereof used for providing the configuration space based on which the treatment plan is determined.

The generated planes shown in Fig. 6D are subsequently shifted in opposite direction to the respective one of the unit vector *̅v̅₁̅*̅, *̅v̅₂̅*̅ to a position outside of the treatment region 60 at which a distance of all points in the respective plane to all points in the treatment region 60, measured in the respective stipulated entry direction 21a, 21b, is equal to or larger than a minimal distance Δ. For instance, distances can be measured based on the meshes of the treatment region 60, which may comprise meshes of the one or more ablation target regions 70 and the one or more risk regions 80. In a vector space having the reference point *̅r̅₀̅*̅ as its origin, for instance, the dot product between vectors corresponding to vertices in the meshes and the unit vector normal to the respective plane can be considered for determining the distances, and thereby the shift of the respective plane. The minimum distance Δ can be a positive internal parameter equal to or larger than zero that can be used to position the planes at any given distance from a body skin surface, for instance, if/when required.

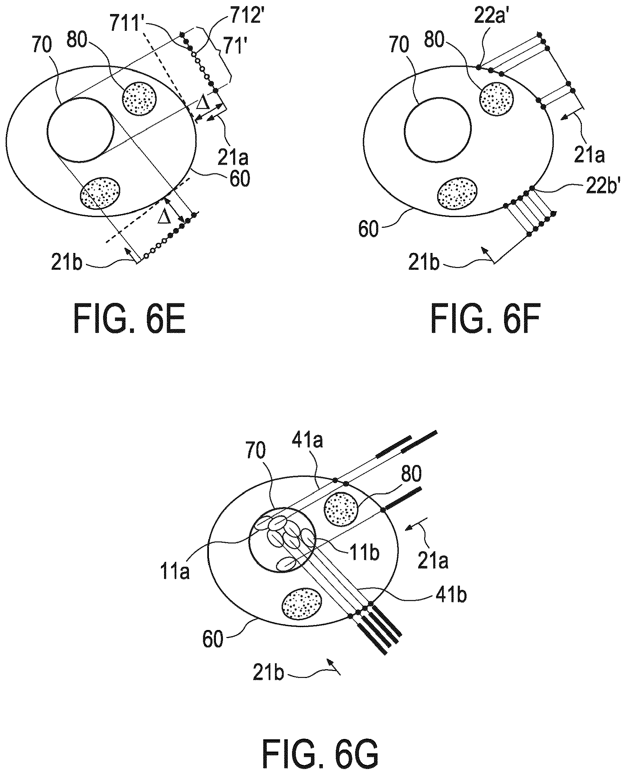

On the shifted planes, planar coordinate systems perpendicular to the respective unit vectors can be established, which are usable to sample projection points based on which the candidate entry points can be determined. To do so, as illustrated in Fig. 6E, the one or more meshes of the one or more ablation target regions 70 are projected on the one or more planes, thereby generating two-dimensional ablation target projections on each of the planes. The two-dimensional ablation target projections can then be used to determine bounding boxes 71' bounding each of the projections on the one or more planes, i.e. fully encompassing the ablation target projections. The determined bounding boxes could also be regarded as fields of view (FoV).

Within each bounding box 71', projection points can be (regularly) sampled with a predefined spacing s in the planar directions of the respective planar coordinate system, thereby generating, for instance, the points 711', 712' indicated in Fig. 6E. Some of these projection points can subsequently be discarded, namely if a candidate trajectory 311a, 311b originating from or passing through them in the respective normal direction of the respective plane would hit a risk region 80. In Fig. 6E, the points which could be discarded for this reason comprise the points 711'. The remaining projection points 712' can be projected on the boundary or surface of the treatment region 60, such as a patient's body skin surface, or a corresponding mesh, to determine the candidate entry points 22a', 22b', as indicated in Fig. 6F. A list of all sampled stipulated entry directions 21a, 21b and corresponding determined candidate entry points 22a', 22b' can then be collected and passed as input for subsequent processing steps, particularly comprising the determination of intersections of the corresponding candidate trajectories 311a, 311b with the ablation target region 70 as described above with reference to Fig. 5.

As already mentioned above, Figs. 6A to 6G do not illustrate that stipulated entry points 22a, 22b are provided as entry conditions. However, similar principles can be applied in order to determine the candidate entry directions 21a', 21b' from stipulated entry points 22a, 22b. For instance, for a given stipulated entry point 22a, 22b, all directed straight lines entering the treatment region 60 through the stipulated entry point 22a, 22b could be considered, wherein from this set of lines those could be identified that pass through one or more ablation target regions 70, particularly without crossing any risk region 80, wherein the remaining identified directed straight lines uniquely specify the candidate entry directions 21a', 21b' for the given stipulated entry point 22a, 22b. The finally generated list of pairs of candidate entry points and candidate entry directions could then contain the stipulated entry point 22a, 22b and the corresponding determined candidate entry directions 21a', 21b'. A stipulated entry point 22a, 22b could be indicated, for instance, by a clinical expert in terms of one or more insertion areas, such as skin insertion areas on a patient's body, wherein the skin insertion areas may be indicated by using a suitable radio-opaque marking pen and the indicated insertion areas can be segmented, for instance, in a medical image, or by indicating a set of virtual skin insertion areas in a three-dimensional rendered image of the treatment region 60, particularly on the imaged patient's body skin. Irrespective of how the insertion areas are indicated, they can be used to sample a set of stipulated entry points 22a, 22b inside the indicated insertion area. For each of the sampled stipulated entry points 22a, 22b, which may particularly be points on a patient's skin, a fan of divergent directions could be simulated and used for the subsequent optimization steps, as mentioned above. Also in case stipulated entry points 22a, 22b are provided, the subsequent processing steps can be carried out in a fully automatic manner in order to identify promising ones among, and/or promising entry points inside, the indicated skin insertion areas.

It is also possible that both stipulated entry points 22a, 22b and stipulated entry directions 21a, 21b are provided. If, for instance, in addition to initially provided stipulated entry points 22a, 22b, stipulated entry directions 21a, 21b are subsequently provided, for each stipulated entry direction 21a, 21b a corresponding one of the divergent directions might be chosen from each fan of directions diverging from the sampled stipulated entry points 22a, 22b. For instance, with *M* ∈ 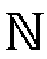 stipulated entry points 22a, 22b being sampled in an indicated insertion area, the fans of divergent directions simulated for each of the sampled stipulated entry points 22a, 22b collectively define an initial set of *M* × *d* candidate trajectories 31 1a, 311b; 312a, 312b if each fan comprises *d* ∈ N directions. If additionally *N* ∈ 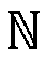 stipulated entry directions 21a, 21b are provided, *N* disjoint subsets of the initial set of *M* × *d* candidate trajectories 311a, 311b; 312a, 312b can be determined, wherein each of the *N* subsets comprises *M* candidate trajectories 311a, 311b that are parallel to one of the stipulated entry directions 21a, 21b, each candidate trajectory in a given subset corresponding to one of the *M* sampled stipulated entry points 22a, 22b and one of the *d* directions in the fan of directions simulated for the respective one of the *M* sampled stipulated entry points 22a, 22b. Hence, from the initial set of *M* × *d* candidate trajectories 311a, 311b; 312a, 312b, subsets of candidate trajectories can be determined, wherein each subset comprises trajectories 311a, 311b that enter the treatment region 60 at the different sampled stipulated entry points 22a, 22b, but do so in a same one of the additionally provided stipulated entry directions 21a, 21b.

Independent of whether stipulated entry directions 21a, 21b or stipulated entry points 22a, 22b are initially provided, the final set of candidate entry directions 21a, 21b and candidate entry points 22a, 22b can be determined, for instance, further based on clinical historical databases, heuristic criteria, etc. Hence, not only criteria like whether a candidate trajectory 311a, 311b; 312a, 312b intersects the ablation target region 70 without intersecting any risk region 80 can be applied. These further criteria can be applied, for instance, in order to single out particular candidate entry directions with corresponding candidate entry points from those following from the procedure described above, as possibly stored in a corresponding, and possibly large, list as indicated above.

Given the final set of candidate trajectories 311a, 311b; 312a, 312b, possibly following from the final set of candidate entry directions and candidate entry points only after considering the intersections of the resulting candidate trajectories 311a, 311b; 312a, 312b with the one or more ablation target regions 70 as described above with reference to Fig. 5 and/or further based on clinical historical databases, and the candidate thermal control parameter values 32i, 32ii possibly corresponding to candidate ablation events along the final candidate trajectories 311a, 311b; 312a, 312b, i.e. based on the final configuration space, an optimized treatment plan can be determined. For this purpose, the candidate thermal impact providing unit 130 may provide thermal impacts characterized by corresponding ablation zones for each of the configurations in the configuration space, wherein then a heuristic mathematical solver serving as the treatment plan determining unit 140 may solve a geometric and combinatorial problem in order to find, among all considered candidate configurations, those configurations that result in an "optimal" plan for the planned thermal ablation treatment.

In order to define the meaning of "optimal", thermal impact objectives may be defined. These may be regarded as mathematical objectives serving as a surrogate for considerations that would otherwise be made by a user. The thermal impact objective to be satisfied by the treatment plan can relate to any of the following: an overall thermal impact on the ablation target region 70, an overall thermal impact on regions outside of the ablation target region 70, a number of activation positions, and/or a geometric relation of activation positions to the ablation target region 70. A single thermal impact objective can relate to several of these or similar objectives, which may then be considered as sub-objectives. In particular, the thermal impact objective can comprise several sub-objectives, wherein the sub-objectives are ranked by priority, and wherein, for determining the treatment plan, candidate treatment plans are compared with respect to the sub-objectives according to their priority. For instance, sub-objectives like a maximum target coverage percentage, a minimum coverage in critical structures, a minimum healthy tissue coverage percentage, a minimum number of ablation zones, and an ablation symmetry (i.e. providing ablation positions that are symmetric to the target segmentations) may be ranked by importance in this order, in which case the optimization problem may be divided into a sequence of single-objective optimization problems, such that during each step of the optimization, treatment plans will be compared based on target coverage first, and in case two solutions are equivalent, these will be compared based on coverage in healthy tissue, particularly critical structures, secondly, etc. A lexicographic set-covering method for multi-objective optimization can be employed, as described in EP 3 777 748 A1 and the Article "A Greedy Heuristic for the Set-Covering Problem" by V. Chvatal, Mathematics of Operations Research, Vol. 4, No. 3, pages 233-235 (1979), which are herewith incorporated by reference in their entirety.

Some of the ablation events corresponding to respective thermal impacts characterized by ablation zones 11a, 11b that could be planned according to a finally determined treatment plan along corresponding planned trajectories 41a, 41b are illustrated in Fig. 6G.

The determined treatment plan satisfying the thermal impact objective can be modified by removing ablation events that can be removed without generating any part in the ablation target region 70 that receives a thermal impact below a predetermined threshold. This may also be referred to as a redundancy check. A redundancy check may be advantageous and further increase overall efficiency although the previous optimization may already aim to select a set of configurations from configuration space that uses as few ablation events as possible, since even after this optimization there may still be redundant ablation events. Within a treatment plan, an ablation event may be considered redundant if its ablation zone within the ablation target region 70 is fully covered by other ablation zones in the same treatment plan. Such redundant ablation events may be present after optimization, because a selected set of trajectories corresponding to a treatment plan may contain one ablation event that is essential for target coverage, while other ablation events in the same plan may be redundant. Therefore, redundant ablation events may be removed from a previously "optimized" treatment plan depending on an outcome of the redundancy check.

The system 100 can be part of an apparatus for thermal ablation treatment planning, wherein the apparatus can further comprise a display device for displaying an image of a treatment region 60, and an input receiving unit for receiving user input with respect to the image, wherein the entry conditions 21a, 21b; 22a, 22b can be provided based on a user input with respect to the image and/or wherein the treatment plan is visualized in the image.

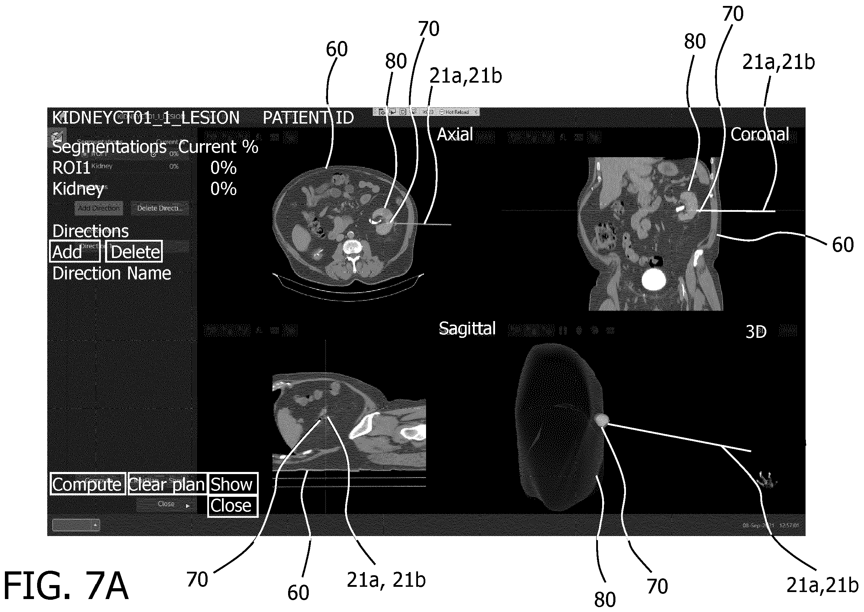

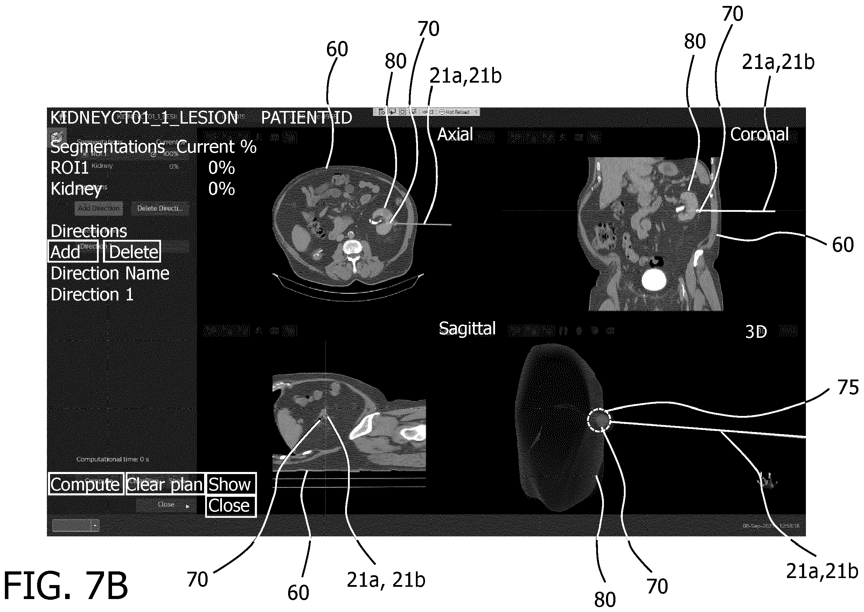

Figs. 7A and 7B show schematically and exemplarily a possible user interface provided on the display of the apparatus, which will subsequently be described in more detail. The user interface can be generated by a corresponding program run on a computer controlling the apparatus. In the illustrated example, the ablation target region 70 corresponds to a kidney tumor. Because the aim of the procedure is to ablate the whole tumor and to spare as much healthy kidney tissue as possible, the kidney itself is, in this case, defined as the organ at risk, i.e. as a risk region 80. On the left tab, some buttons are provided to, for instance, add a stipulated entry direction 21a, 21b ("Add Direction"), to delete a stipulated entry direction 21a, 21b ("Delete Direction"), to compute a treatment plan ("Compute"), and to clear a treatment plan ("Clear Plan").

The very first planning step consists of providing the list of stipulated entry directions 21a, 21b, i.e. of three-dimensional (3-D) directions to use for computing the optimal treatment plan (Fig. 7A). This is done via clicking the "Add Direction" button and by iteratively clicking two points on the displayed 3-D medical image views (axial, coronal and sagittal) for each stipulated entry directions 21a, 21b. The user can provide stipulated entry directions 21a, 21b by freely clicking the two points in-plane over the same image slice, and/or also over different slices. In the tab on the left, it is possible to observe the dynamically populated list of stipulated entry directions 21a, 21b progressively added by the user.

Once all stipulated entry directions 21a, 21b are properly provided, the user can run an ablation plan optimization by simply clicking on the "Compute" button (Fig. 7B). Finally, the set of optimal ellipsoidal ablation zones 75 parallel to the given stipulated entry directions 21a, 21b and covering the ablation target region are shown. The ablation zones 75 correspond to optimally delivered thermal impacts. The ablation coverage percentages for all segmented regions are displayed in the top-left corner of the user interface, next to the names of the employed meshes corresponding to the ablation target region 70 ("ROI 1") and the risk region 80 ("Kidney").

A user may indicate the stipulated entry directions 21a, 21b differently than by subsequently clicking points on a medical image as described above with respect to Figs. 7A and 7B. For instance, a user may also indicate the stipulated entry directions 21a, 21b via a tracking device ahead of applicator placement. The tracking device may be an optical or an electro-magnetic tracking device, for instance. The stipulated entry direction 21a, 21b indicated in this way may be detected by the apparatus as described above and could then be visualized in real-time on the user interface as seen in Fig. 7A and 7B. Then, a mathematical solver corresponding the treatment plan determining unit 140 may determine an optimal treatment plan, and the user can decide whether to continue with the plan execution, or to try again by proposing a new insertion direction as stipulated entry direction 21a, 21b.

Stipulated entry directions 21a, 21b could be indicated by only clicking a single point on the user interface. The second point required to depict a vector corresponding to the respective stipulated entry direction 21a, 21b may then be automatically computed based on provided anatomical segmentations of the treatment region 60. For example, as second point an ablation target region centroid, or even the geometrical barycenter of all ablation target region centroids could be used.

Every time the user modifies, adds and/or removes one or more stipulated entry directions 21a, 21b, automatically a corresponding new treatment plan may be re-computed and displayed on the user interface.

The user can provide a set of stipulated entry directions 21a, 21b via pre-implanted devices, i.e. devices implanted in the treatment region 60 in preparation of the actual treatment planning, that can be properly segmented out of clinical images. A mathematical solver corresponding to the treatment plan determining unit 140 can then constrain the plan optimization so that all returned optimal ablation events will be positioned along those placed device tracks.

The system 100 can also work in a fully automated manner. A corresponding automatic method carried out by the system 100 could comprise computing a set of optimal entry directions 21a, 21b based on a set of clinical criteria, and/or clinical knowledge, and/or a historical database of treatment plans pre-optimized by clinical experts. Potentially, a user could additionally provide the otherwise automatically working system 100 with some additional direction hints as described in the previous semi-automatic cases. However, the automatic algorithm can be totally free to select other, different insertion directions 21a, 21b if/when considered more promising as compared to the ones advised by the user.

Particularly in case of fully automatized treatment planning, the system 100 can further comprise an artificial intelligence providing unit for providing an artificial intelligence that has been trained to provide, i.e. as output, output entry conditions of thermal ablation treatment plans satisfying a thermal impact objective if it is provided, i.e. as input, with a three-dimensional input image of an input region comprising an input ablation target region, wherein the entry condition providing unit 110 can be configured to provide the artificial intelligence, i.e. as input, with a three-dimensional image of the treatment region 60 comprising the thermal ablation target region 70 to be treated, and to provide, as the entry conditions 21a, 21b; 22a, 22b to be used for the treatment to be planned, the output entry conditions provided, i.e. as output, by the artificial intelligence. Hence, for instance, the artificial intelligence could be trained to receive a medical image as shown in Fig. 7A as an input and to provide an entry direction 21a, 21b as indicated in Fig. 7A that can lead to a treatment plan satisfying a predefined thermal impact objective as an output.

The artificial intelligence can particularly comprise a convolutional neural network (CNN) trained to infer a set of optimal insertion directions for a given patient of the day. The CNN model training could be performed using a historical database collecting a large amount of optimal treatment directions used in true clinical plans, i.e. treatment plans that have actually been followed in the past clinical practice. Here, each database dataset could store the clinical 3-D images, and/or the segmented anatomical regions, and the 3-D direction unit vectors' components of the set of executed treatment devices, i.e. ablation applicators. The clinical images can be passed via an input layer of the CNN, and the CNN model can be trained to then infer and return as output the related optimal direction unit vectors. The training and/or tuning of the model could be executed by splitting the clinical database datasets in two separate learning and training sets, or even in training, validation and test data sets. A training system can be used for training the artificial intelligence based on past treatment plans as training data. The system 100 itself can function as such a training system, wherein it may then comprise technical means configured for receiving the training input and training output data.

The entry condition providing unit 110 can be configured to a) determine a length of intersection of at least a subset of candidate trajectories 311a, 311b; 312a, 312b entering the treatment region 60 according to at least a subset of all possible entry conditions with the ablation target region 70, wherein the entry conditions 21a, 21b; 22a, 22b are provided based on the determined lengths of intersection.

Additionally or alternatively, the entry condition providing unit 110 can be configured to b) determine an ablation target distance for at least a subset of candidate trajectories 311a, 311b; 312a, 312b entering the treatment region 60 according to at least a subset of all possible entry conditions, the ablation target distance being a length of the respective candidate trajectories 311a, 311b; 312a, 312b from entering the treatment region 60 to reaching the ablation target region 70, wherein the entry conditions 21a, 21b; 22a, 22b are provided based on the ablation target distances.

In particular, the set of optimal entry directions could be automatically selected, such as from an automatically generated, possibly relatively large but discrete, number of 3-D directions, for instance, wherein the selection could be based on a set of heuristic selection criteria comprising criteria corresponding to following options a) and/or b).

According to option a), for each candidate entry direction a discrete number of intersection segments corresponding to segments of candidate trajectories 311a, 311b; 312a, 312b having the candidate entry direction and passing through the ablation target region 60 could be sampled and computed. The longer the intersection segments are the better, since trajectories with larger intersection segments allow to cover larger portions of the ablation target region 60.

According to option b), for each candidate entry direction 3-D distances from the skin body surface to ablation target region points (e.g., its mesh vertices) could be computed along the candidate direction. The average, or minimum, distance value computed for each candidate direction could be used as criterion to select the one or more optimal entry directions. In particular, the smaller the target-to-skin average distance is, the shorter may be the path that needs to be taken by the thermal ablation applicator comprising the thermal ablation source 50 in order to reach the thermal ablation target for treatment, and/or the larger may be the portion of the thermal ablation target that can be reached with the thermal ablation applicator.

Again, direction unit vectors could be shown to a clinical expert or other user on a user interface as illustrated by Figs. 7A and 7B, possibly with a color-coded scale indicating the respective selection criterion value, or a composite selection criteria value.

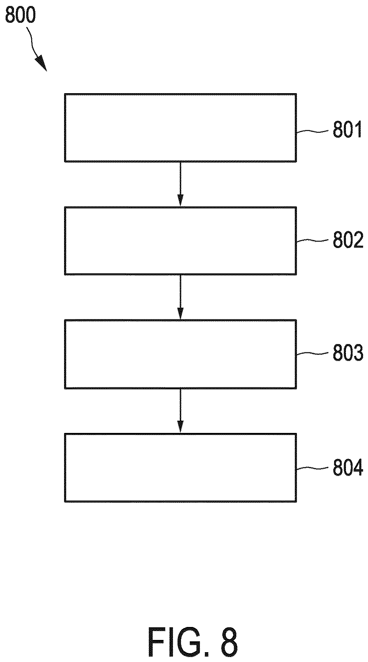

Fig. 8 shows schematically and exemplarily a method 800 for thermal ablation treatment planning, wherein the method 800 comprises a step 801 of providing an entry condition 21a, 21b; 22a, 22b indicative of how a trajectory of a thermal ablation source 50 to be planned is to enter a treatment region 60 comprising an ablation target region 70, a step 802 of providing a configuration space comprising, as configurations, a) candidate positions corresponding to a plurality of candidate trajectories 311a, 311b; 312a, 312b of the thermal ablation source 50 through the treatment region 60 and b) candidate thermal control parameter values 32i, 32ii for each of the candidate positions, wherein the candidate trajectories 311a, 311b; 312a, 312b enter the treatment region 60 in accordance with the provided entry condition 21a, 21b; 22a, 22b, and a step 803 of providing candidate thermal impacts 10a, 10b generated by the thermal ablation source 50 on its neighborhood in each of the configurations. The method 800 further comprises a step 804 of determining a thermal ablation treatment plan comprising a) a planned trajectory 41a, 41b of the thermal ablation source 50 and b) planned thermal control parameter values along the planned trajectory 41a, 41b, wherein the treatment plan is determined based on the candidate thermal impacts 10a, 10b provided for each of the configurations such that a predetermined thermal impact objective is satisfied by the treatment plan.

The method 800 can be implemented, for instance, by executing a computer program for thermal ablation treatment planning that comprises program code means for causing the system 100 to carry out the method 800.

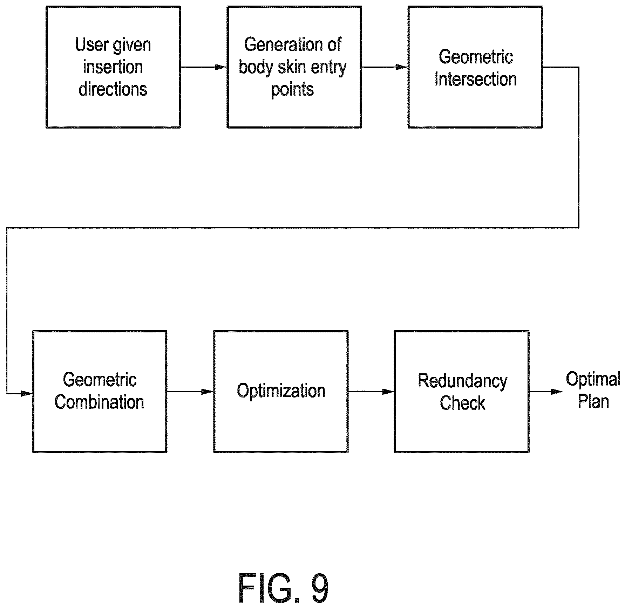

Fig. 9 shows schematically and exemplarily a flowchart summarizing particular aspects of the method 800, which can be executed by the system 100, wherein several steps are grouped in the form of blocks, which may be considered as representing functionally and/or logically separable stages of the method 800. Given, as an input, a set of provided insertion directions providable as stipulated entry directions 21a, 21b by a user, a proposed "optimal" thermal ablation treatment plan to be followed using a given ablation device may be arrived at as an output, wherein also all possible ablation zones may be considered in arriving at the output.

In a first stage, which has been exemplarily described with reference to Figs. 6A to 6F, body skin entry points are generated in order to identify the set of skin entry points 22a', 22b' to be used for inserting all medical devices. This can involve computing a set of planes perpendicular to the provided directions 21a, 21b, computing a set of bounding boxes on each plane encompassing the target projections, sampling a set of points in the respective planes within each bounding box, projecting the sampled points on the patient body skin surface, and collecting a list of entry trajectories 311a, 311b corresponding to pairs of skin entry points 22a', 22b' and provided directions 21a, 21b.

In a second stage, which may be referred to as "Geometric Intersection", all potential applicator trajectories that intersect with the target 70 may be identified, which may involve computing a list of target intersecting segments from the list of entry trajectories 311a, 311b, as exemplarily described above particularly with reference to Fig. 5.

In a third stage, which may be referred to as "Geometric Combination", all potential configurations (combined ablations) along each remaining trajectory may be defined, which may involve computing a table of ablation shape configurations for each target segment. This has been exemplarily described with reference to Figs. 4A to 4F, wherein information as exemplarily explained with reference to Fig. 3 may be used for "Geometric Combination".

In a fourth stage, which may be referred to as "Optimization", an optimal combination of previously calculated configurations may be selected, which may involve computing organ volume coverage percentages for each table configuration, and solving a mathematical set covering problem to find the optimal set of ablation configurations satisfying all clinical protocol goals set by the clinical expert. Known approaches may be used at this stage.

In a fifth stage, a "Redundancy Check" can be carried out in order to prevent unnecessary ablations, which may, as indicated above, involve removing redundant ablations.

The result of this five-stage process may be an optimal thermal ablation treatment plan. The whole process can be implemented as a planning algorithm that can be controlled via a user interface, wherein a user may be asked to delineate a set of insertion directions 21a, 21b using suitable visualization tools. During the five stages, all provided inputs may be processed to generate internal objects and data that may allow an inner mathematical solver to identify and return as one output the best treatment plan to deliver. Particular realizations of the process illustrated in Fig. 9 have been described above in more detail. In particular, different ways of determining optimal skin entry points 22a', 22b' and insertion path directions 21a', 21b' at the first stage have been described. The remaining stages, i.e. stages two to five, can be realized in a same manner irrespective of how the first stage is realized.

Thermal ablation cancer treatments are getting increasingly popular due to their applicability to non-resectable tumors and the rapid recovery of the patient. However, thermal ablation planning tools are significantly immature.

Thermal ablation treatment planning concerns the optimal placement of the ablation devices, which is often determined based on the tumor position and size, the device manufacturers' specifications, and the physician's experience. Moreover, in order to reduce unwanted damage to nearby healthy tissues, the overall ablated region during a treatment should conform as much as possible to the tumor contours, wherein oncological safety margins may need to be respected. Often, thermal ablation treatment procedures require the physician to choose a set of probe positions, i.e. positions of an applicator device and/or particular a thermal ablation source thereon, and a corresponding set of delivery parameters, which may be concluded from ablation power and time values given in the probe manufacturer's specifications. To support the probe positioning, rigid leading grid templates, such as those also used in brachytherapy, or flexible radio-opaque grids can be used to guide and lead the insertion of all treatment devices inside the patient body.

However, for some cancer types, like in renal cancer thermal ablation treatments, no leading grid templates are used for guidance, but the treatment is executed in a freehand manner. In this case, based on medical images of all cancer lesions, and nearby risk organs, ureter(s), colon, vessels, nerves, muscles, etc., the clinical expert needs to decide on a limited set of skin entry points and probe needle 3-D insertion tracks, and directions to follow in order to reach and ablate all target cancer lesions. In addition to solving the device positioning problem, the expert is also confronted with the problem to select the best device ablation settings to use for treatment. This manual forward planning procedure can be very lengthy and error-prone. Overall, the numerous degrees of freedom of particularly the grid-less thermal ablation treatment planning problem makes its inherent space of possible solutions quite large, and a manual selection of the optimal plan could easily miss the best possible solution. It has been realized, therefore, that improved means, possibly implemented in the form of a mathematical solver, for searching for the best plan (i.e. the best set of skin entry trajectories and device ablation settings) could be beneficial to simplify the planning process, to increase the plan coverage quality, to make the whole planning process more standardized, and to make the whole planning process more efficient. It was then found that such means can be realized by a thermal ablation therapy planning system as described herein, particularly functioning based on a provided set of cancer lesions and other anatomical structure segmentations as well as associated prescriptions (i.e., protocol objectives), a provided set of ablation settings of one or more commissioned devices usable for treatment, and a provided set of 3-D insertion conditions, particularly directions, possibly provided by the user via a suitable user interface, wherein then an optimal treatment plan may be computed consisting of a set of treatment devices to be inserted along trajectories parallel to at least one of the given input directions.

One or more treatment devices comprising respective thermal ablation sources can be used in a treatment. The one or more trajectories corresponding to a treatment plan may be followed, as mentioned further above, sequentially by one or more of the devices, or may be substantially followed simultaneously, wherein it may particularly not be necessary to remove devices while others are inserted into the treatment region.

Although in the embodiments described above the treatment region only includes one ablation target region, in other embodiments the treatment region may include any number, particularly more than one ablation target region. Further configurations may then be considered based on the further one or more ablation target region, such that the determined treatment plan may cover all ablation target regions in the treatment region.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like providing an entry condition, providing a configuration space, providing a thermal impact and determining a thermal ablation treatment plan, etc., performed by one or several units or devices, can be performed by any other number of units or devices. These procedures can be implemented in terms of program code means of a computer program and/or in terms of dedicated hardware.

A computer program may be stored/distributed on a suitable medium, such as an optical storage medium or a solid-state medium, which may be supplied together with or as part of other hardware, but may also be distributed in other forms, such as via the Internet or other wired or wireless telecommunication systems.

Any reference signs in the claims should not be construed as limiting the scope.

A system for thermal ablation treatment planning is provided that allows to provide an entry condition indicative of how a trajectory of a thermal ablation source is to enter a treatment region. A configuration space is then provided that comprises, as configurations, a) candidate positions corresponding to candidate trajectories entering the treatment region in accordance with the provided entry condition, and b) candidate thermal control parameter values for each of the candidate positions. Based on candidate thermal impacts provided for each of the configurations, a treatment plan is determined that satisfies a thermal impact objective.

## Claims

1. A system (100) for thermal ablation treatment planning, wherein the system comprises:
- an entry condition providing unit (110) configured to provide an entry condition (21a, 21b; 22a, 22b) indicative of how a trajectory of a thermal ablation source (50) to be planned is to enter a treatment region (60) comprising an ablation target region (70),
- a configuration space providing unit (120) configured to provide a configuration space comprising, as configurations, a) candidate positions corresponding to a plurality of candidate trajectories (311a, 311b; 312a, 312b) of the thermal ablation source (50) through the treatment region (60) and b) candidate thermal control parameter values (32i, 32ii) for each of the candidate positions, wherein the candidate trajectories (311a, 311b; 312a, 312b) enter the treatment region (60) in accordance with the provided entry condition (21a, 21b; 22a, 22b),
- a candidate thermal impact providing unit (130) configured to provide candidate thermal impacts (10a, 10b, 10c, 10d) generated by the thermal ablation source (50) on its neighborhood in each of the configurations, and
- a treatment plan determining unit (140) configured to determine a thermal ablation treatment plan comprising a) a planned trajectory (41a, 41b) of the thermal ablation source (50) and b) planned thermal control parameter values along the planned trajectory (41a, 41b), wherein the treatment plan is determined based on the candidate thermal impacts (10a, 10b, 10c, 10d) provided for each of the configurations such that a predetermined thermal impact objective is satisfied by the treatment plan.

2. The system as defined in claim 1, wherein the entry condition providing unit (110) is configured to provide a plurality of entry conditions (21a, 21b; 22a, 22b), wherein the configuration space providing unit (120) is configured to provide the configuration space such that the candidate trajectories (311a, 311b; 312a, 312b) each enter the treatment region (60) in accordance with one of the entry conditions (21a, 21b; 22a, 22b), wherein the treatment plan determining unit (140) is configured to determine the treatment plan such that it comprises a) a plurality of planned trajectories (41a, 41b) to be followed during treatment and b) planned thermal control parameter values along the planned trajectories (41a, 41b).

3. The system as defined in claim 1 or 2, wherein the entry conditions (21a, 21b; 22a, 22b) each comprise a stipulated entry direction (21a, 21b), wherein for each of the stipulated entry directions (21a, 21b), a plurality of candidate entry points (22a', 22b') at which the one or more candidate trajectories (311a, 311b) entering the treatment region (60) in the stipulated entry direction (21a, 21b) do so are determined based on the stipulated entry direction (21a, 21b) and the treatment region (60).

4. The system as defined claim 1 or 2, wherein the entry conditions (21a, 21b; 22a, 22b) each comprise a stipulated entry point (22a, 22b), wherein for each of the stipulated entry points (22a, 22b), a plurality of candidate entry directions (21a', 21b') in which the one or more candidate trajectories (312a, 312b) entering the treatment region (60) at the stipulated entry point (22a, 22b) do so are determined based on the stipulated entry point (22a, 22b) and the treatment region (60).

5. The system as defined in any of the preceding claims, wherein the candidate thermal control parameter values (32i, 32ii) along the candidate trajectories (311a, 311b; 312a, 312b) are chosen such that the thermal ablation source (50) is only activated inside the ablation target region (70).

6. The system as defined in any of the preceding claims, wherein the candidate thermal control parameter values (32i, 32ii) indicate activation positions along the respective candidate trajectory (311a, 311b; 312a, 312b) at which the thermal ablation source (50) is activated and a candidate thermal impact (10a, 10b) of the activated thermal ablation source (50) at the respective activation positions.

7. The system as defined in any of the preceding claims, wherein the candidate thermal control parameter values (32i, 32ii) indicate a fixed set of activation positions along each of the candidate trajectories (311a, 311b; 312a, 312b) and/or a fixed set of candidate thermal impacts (10a, 10b, 10c, 10d).

8. The system as defined in any of the preceding claims, wherein the thermal impact objective to be satisfied by the treatment plan relates to any of an overall thermal impact on the ablation target region (70), an overall thermal impact on regions outside of the ablation target region (70), a number of activation positions, and/or a geometric relation of activation positions to the ablation target region (70).

9. The system as defined in any of the preceding claims, wherein the thermal impact objective comprises several sub-objectives, wherein the sub-objectives are ranked by priority, and wherein, for determining the treatment plan, candidate treatment plans are compared with respect to the sub-objectives according to their priority.

10. The system as defined in any of the preceding claims, wherein the determined treatment plan satisfying the thermal impact objective is modified by removing ablation events that can be removed without generating any part in the ablation target region (70) that receives a thermal impact below a predetermined threshold.

11. The system as defined in any of the preceding claims, wherein the system further comprises an artificial intelligence providing unit for providing an artificial intelligence that has been trained to provide output entry conditions of thermal ablation treatment plans satisfying a thermal impact objective if it is provided with a three-dimensional input image of an input region comprising an input ablation target region, wherein the entry condition providing unit is configured to provide the artificial intelligence with a three-dimensional image of the treatment region (60) comprising the thermal ablation target region (70) to be treated, and to provide, as the entry conditions (21a, 21b; 22a, 22b) to be used for the treatment to be planned, the output entry conditions provided by the artificial intelligence.

12. The system as defined in any of the preceding claims, wherein the entry condition providing unit (110) is configured to:
a) determine a length of intersection of at least a subset of candidate trajectories (311a, 311b; 312a, 312b) entering the treatment region (60) according to at least a subset of all possible entry conditions with the ablation target region (70), wherein the entry conditions (21a, 21b; 22a, 22b) are provided based on the determined lengths of intersection, and/or wherein the entry condition providing unit (110) is configured to:
b) determine an ablation target distance for at least a subset of candidate trajectories (311a, 311b; 312a, 312b) entering the treatment region (60) according to at least a subset of all possible entry conditions, the ablation target distance being a length of the respective candidate trajectory (311a, 311b; 312a, 312b) from entering the treatment region (60) to reaching the ablation target region (70), wherein the entry conditions (21a, 21b; 22a, 22b) are provided based on the ablation target distances.

13. An apparatus for thermal ablation treatment planning, wherein the apparatus comprises:
- a display device for displaying an image of a treatment region (60),
- an input receiving unit for receiving user input with respect to the image, and
- the system (100) as defined in to any of claims 1 to 12,
wherein the entry condition providing unit (110) is configured to provide the entry conditions (21a, 21b; 22a, 22b) based on a user input with respect to the image and/or wherein the display device is configured to visualize the treatment plan in the image.

14. A method (800) for thermal ablation treatment planning, wherein the method comprises:
- providing (801) an entry condition (21a, 21b; 22a, 22b) indicative of how a trajectory of a thermal ablation source (50) to be planned is to enter a treatment region (60) comprising an ablation target region (70),
- providing (802) a configuration space comprising, as configurations, a) candidate positions corresponding to a plurality of candidate trajectories (311a, 311b; 312a, 312b) of the thermal ablation source (50) through the treatment region (60) and b) candidate thermal control parameter values (32i, 32ii) for each of the candidate positions, wherein the candidate trajectories (311a, 311b; 312a, 312b) enter the treatment region (60) in accordance with the provided entry condition (21a, 21b; 22a, 22b),
- providing (803) candidate thermal impacts (10a, 10b, 10c, 10d) generated by the thermal ablation source (50) on its neighborhood in each of the configurations, and
- determining (804) a thermal ablation treatment plan comprising a) a planned trajectory (41a, 41b) of the thermal ablation source (50) and b) planned thermal control parameter values along the planned trajectory (41a, 41b), wherein the treatment plan is determined based on the candidate thermal impacts (10a, 10b, 10c, 10d) provided for each of the configurations such that a predetermined thermal impact objective is satisfied by the treatment plan.

15. A computer program for thermal ablation treatment planning, wherein the program comprises program code means for causing the system (100) as defined in any of claims 1 to 12 to execute the method (800) as defined in claim 14.
